# EUROPEAN PATENT SPECIFICATION

(11) **EP 2 943 230 B1**
(45) Date of publication and mention of the grant of the patent: **13.12.2017**
(21) Application number: 14703419.3
(22) Date of filing: 02.01.2014
(51) Int. Cl.: A61L 27/18, A61L 27/38

(54) **A POLYMERIC SCAFFOLD FOR CARDIAC REGENERATION AND PROTECTION FROM REPERFUSION INJURY**
POLYMERGERÜST FÜR KARDIALE REGENERIERUNG UND SCHUTZ VOR REPERFUSIONSSCHÄDEN
ÉCHAFAUDAGE POLYMÈRE POUR RÉGÉNÉRATION CARDIAQUE ET PROTECTION CONTRE UNE LÉSION DE REPERFUSION

(30) Priority: 09.01.2013 IT TO20130014
(43) Date of publication of application: 18.11.2015
(73) Proprietor: Università Degli Studi Di Torino, 10124 Torino (IT); Università di Pisa, 56126 Pisa (IT)
(72) Inventor: CRISTALLINI, Caterina, I-56126 Pisa (IT); GIACHINO, Claudia, I-10121 Torino (IT); BARBANI, Niccoletta, I-56126 Pisa (IT); CIBRARIO ROCCHIETTI, Elisa, I-10045 Piossasco (Torino) (IT); GAGLIARDI, Mariacristina, I-56126 Pisa (IT); PAGLIARO, Pasquale, I-10139 Torino (IT)
(74) Representative: Freyria Fava, Cristina
(86) International application number: PCT/IB2014/058025
(87) International publication number: WO 2014/108814

(56) References cited:
- WO-A1-03/032867
- WO-A2-2008/106485
- D.-H. KIM ET AL: "Nanoscale cues regulate the structure and function of macroscopic cardiac tissue constructs", PROCEEDINGS OF THE NATIONAL ACADEMY OF SCIENCES, vol. 107, no. 2, 12 January 2010 (2010-01-12), pages 565-570, XP055081377, ISSN: 0027-8424, DOI: 10.1073/pnas.0906504107

## Description

The present invention relates in general to a device for use in tissue engineering. Particularly, the invention relates to a polymeric scaffold that, in virtue of its particular geometry and chemical composition, is demonstrated to be highly effective in the regeneration of cardiac tissue. In preferred embodiments, the scaffold of the invention is also provided with cardioprotective ability against damage caused by reperfusion.

The greatest criticality in the treatment of cardiovascular diseases is the restoration of impaired cardiac functions as a result of a heart attack.

The impairment of cardiac activity is linked to the acute phase of the event of infarction, with consequent tissue damage at the level of the ischemic zone, and subsequently to the poor regenerative capacity of the myocardial tissue.

To reduce the initial ischemic injury, techniques of reperfusion are adopted, but reperfusion itself can cause damage to the cells that survive the initial ischemic event. Different studies have indeed shown that so-called reperfusion injuries can represent more than 50% of the lesion size of the post-infarction cardiac tissue. For this reason, the development of strategies to protect the post-ischemic myocardium and maximize the benefits of reperfusion represents one of the most important targets of research in the cardiovascular field.

Tissue engineering is emerging as a potentially new therapeutic approach for the regeneration of damaged myocardium. Different strategies have been attempted in order to achieve this result. For example, several research groups have proposed the *ex vivo* culture of cardiac cells on biodegradable polymeric materials, subsequently implantable *in vivo.* However, this approach poses several problems and limitations, among which the limited proliferative capacity of the cardiomyocytes is especially evident.

A few years ago, a population of multipotent stem cells residing in the adult heart was characterized, which seemed very promising for the use in cardiac regenerative medicine. An interesting perspective for the regeneration of cardiac tissue would therefore be the engineering of support structures (hereinafter referred to as "scaffolds") made of biocompatible polymeric material, able to control the differentiation of stem cells toward a cardiovascular phenotype, modulating the spatial environment of the cells with biochemical and physical signals in order to prevent myocardial dysfunction.

As an alternative strategy to the *ex vivo* culture of cardiac cells, the use of scaffolds made of biocompatible and biodegradable polymeric material for implantation in the infarcted regions was therefore proposed, and in particular on the myocardial wall through an external suture. These scaffolds would play a dual role: on the one hand, they would act as a mechanical support for the damaged cardiac tissue and on the other hand, they would favor the adhesion and proliferation of cardiac cells and their precursors. The paper "Nanoscale cues regulate the structure and function of macroscopic cardiac tissue constructs", Proceedings of the National Academy of Sciences, vol. 107, no. 2, 12 January 2010 (2010-01-12), pages 565-570, discloses a study showing the possibility to reconstruct heart tissue by culturing myocytes on PEG nanopatterned substrate. The substrate presents grooves and ridges to mimic the natural myocardial ECM and thus allows for cells orientation. The recesses have a depth between 0.2 and 0.5 µm, the ridges and the grooves have a width between 0.15 and 0.8 µm. In general, suitable biomaterials for use in tissue engineering should meet the following requirements: be biocompatible and bioactive in order to facilitate cell adhesion and differentiation and, in general, the total integration of the implant without triggering an immune or inflammatory response by the organism; be biodegradable and bioresorbable, with a controlled degradation and a degree of reabsorption such as to allow the growth of cells/tissues *in vitro* and/or *in vivo;* possess appropriate physico-chemical properties; possess mechanical properties as similar as possible to those of the tissue that are found at the site of implantation; be mechanically stable for an appropriate time, in order to maintain the three-dimensional structure until the formation of new biological tissue.

In particular, engineered cardiac tissue should present the same functional properties of the natural cardiac muscle tissue and should also remain viable even after the implant. The mechanical, electrical and functional integration into the architecture of the organ should also improve systolic and diastolic function of the infarcted myocardium. The scaffolds should therefore be contractile, electrophysiologically stable, mechanically robust but flexible, suitable for vascularization and non-immunogenic.

In recent years, several types of polymers, both natural and synthetic, have been studied for use in the engineering of cardiac tissue. The natural polymers described for this purpose in the prior art include collagen, gelatin and alginate. Among the synthetic polymers, several studies have been carried out on polyesters such as polylactic acid (PLA), polyglycolic acid (PGA), polycaprolactone (PCL) and polyurethanes. It has been observed, however, that after repeated stress due to cardiac cycles, the polyesters may undergo plastic deformation.

In addition to the properties mentioned previously, a scaffold suitable for cardiac regeneration must also meet the following requirements: possess high porosity with a network of interconnected pores, in order to allow both the growth of cells, and nutrient transport and catabolite elimination; be easily producible in different shapes and sizes, in order to allow industrialization of the production process; be easily sterilizable with methods that do not modify the structure and chemical composition. It has also been observed that the geometry of the microstructure and the size of the pores of the scaffolds should be specifically studied and selected according to the cell line in which proliferation is required. For example, it was found that the cellular adhesion of cardiomyocytes is favored by structures with a honeycomb morphology with pores having a width comparable to the size of these cells.

The same inventors of the present patent application have studied polymeric scaffolds of a rectangular microstructure according to a predefined geometry designed to mimic the anatomical features of the myocardium. However, such scaffolds did not prove to be effective in facilitating the proliferation and differentiation of cardiac stem cells toward the cardiac lineage, confirming that microstructuring alone is not sufficient to ensure cardioinductivity.

Despite numerous studies and intense research efforts carried out in the field of tissue engineering and in particular of cardiac tissue, the devices produced so far have several functional limitations and do not possess all the qualities required for clinical use as myocardium substitutes.

There is therefore the need to provide new polymeric scaffolds for the regeneration of cardiac tissue that overcome the problems of the prior art.

According to the present invention, this and other objects are achieved by means of a polymeric scaffold for cardiac regeneration which comprises a support structure comprising at least one support layer, the support layer being characterized in that it is made of a polymeric material selected from the group consisting of poly(lactic-co-glycolic) acid, poly(3-hydroxybutyric-co-3-hydroxyvaleric) acid and poly(dioxanone), possibly in association with a biological polymer, the support layer being further characterized in that it has a surface on which a plurality of cavities, arranged in a regular lattice, is formed, said cavities being defined by an array of longitudinal linear projections and by an array of transverse linear projections, respectively intersecting each other.

Thanks to this combination of geometric features and chemical composition, the polymeric scaffold of the invention has been shown to be effective in the regeneration of cardiac tissue, as demonstrated by the experiments conducted by the inventors, which are illustrated in the experimental part of the present description and specifically in the section relative to the biological evidence.

Further characteristics and advantages of the invention are explained in greater detail in the following detailed description, provided purely by way of non-limiting example and with reference to the attached drawings, wherein:
- Figure 1a is a top view of one embodiment of the polymeric scaffold of the invention;
- Figure 1b is a cross section of the polymeric scaffold of Figure 1a;
- Figure 1c is a longitudinal section of the polymeric scaffold of Figure 1a;
- Figure 2 is a cross sectional view of a further embodiment of the polymeric scaffold of the invention.

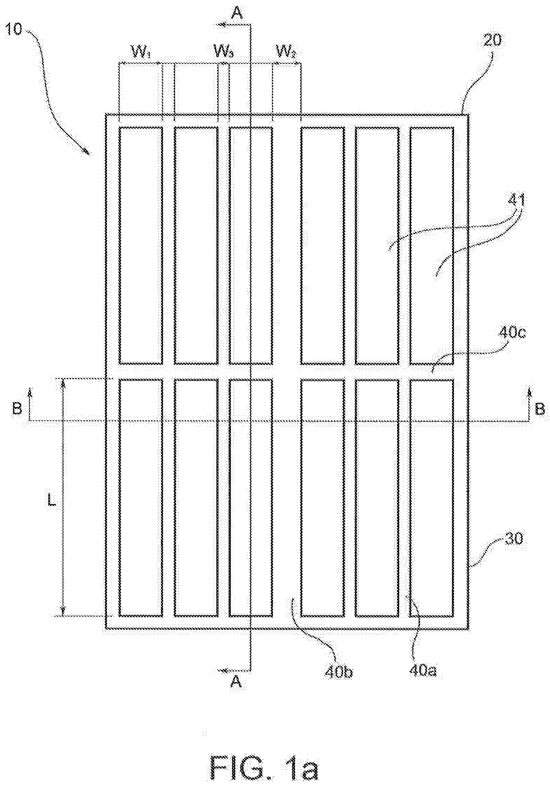

In Figure 1a, the reference number 10 indicates a polymeric scaffold for cardiac regeneration according to the invention, which comprises a support structure indicated with the reference number 20. The support structure 20 in turn comprises at least one support layer 30 made of polymeric material, which presents particular geometric characteristics and of a chemical composition which renders the scaffold 10 surprisingly effective in the regeneration of cardiac tissue. Concerning the chemical composition, the support layer 30 is characterized by a precise selection of the constituent polymers, which are selected from poly(lactic-co-glycolic) acid (PLGA), poly(3-hydroxybutyric-co-3-hydroxyvaleric) acid (PHBHV) and poly(dioxanone) (PDS), possibly in combination with a biological polymer which is preferably selected from the group consisting of gelatin, hyaluronic acid, collagen and gellan gum. Concerning the geometrical characteristics, the support layer 30 is characterized by the presence of a plurality of cavities 41 formed on one of its surfaces or faces, wherein cavities 41 are arranged in a regular lattice and are defined by an array of longitudinal linear projections 40a, 40b and by an array of transverse linear projections 40c, respectively intersecting each other.

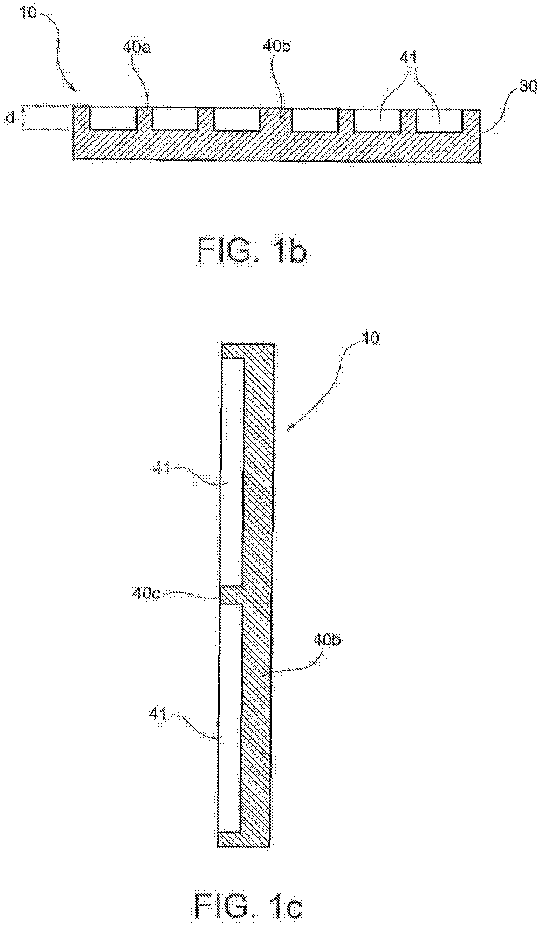

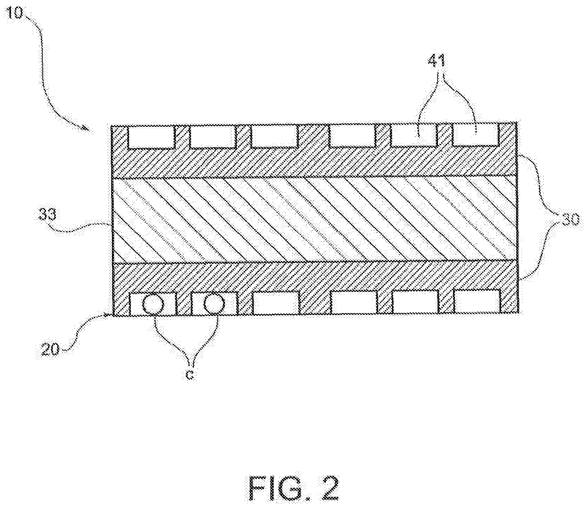

According to an embodiment specifically represented in Figures 1a-1c, the cavities 41 have a longitudinal dimension and a transverse dimension, which in Figure 1a are indicated with L and W₁, respectively. Preferably, the cavities 41 have a longitudinal dimension L in the range of 350-600 µm and a transverse dimension W₁ in the range of 30-150 µm, but these values should not be interpreted in a limiting sense in any way. Figure 1b (which represents the transverse section along the plane B-B of the embodiment of the polymeric scaffold 10 of Figure 1a) also shows that the cavities 41 are characterized by a depth d, which is preferably, but not limited to, in the range of 20-100 µm. These dimensions are preferred as they readily allow the housing of cardiac stem cells (indicated in Figure 2 by C).

According to the embodiment specifically illustrated in Figures 1a-1c, the cavities 41 essentially have a rectangular shape. However in the context of the invention, alternative embodiments apply, which are not specifically illustrated in the figures, wherein the cavities 41 have different geometric shapes, which can be essentially regular or even irregular.

As is clearly shown in Figure 1a, the cavities 41 are defined by an array of longitudinal linear projections 40a, 40b and by an array of transverse linear projections 40c, respectively intersecting each other. The longitudinal linear projections 40a, 40b are also shown in Figure 1b. The transverse linear projections 40c are also indicated in Figure 1c, which represents the transverse section along the plane A-A of the embodiment of the polymeric scaffold 10 of Figure 1a.

According to the embodiment illustrated in Figure 1a, there are two different types of longitudinal projections: wide projections 40b and narrow projections 40a, which are arranged in an alternating sequence. The wide projections 40b are characterized by a transverse dimension W₂ which is preferably (but not limited to) in the range of 50-160 µm; the narrow projections 40a are characterized by a transverse dimension W₃ which is preferably (but not limited to) in the range of 20-120 µm, with the proviso that W₂ is greater than W₃. In the embodiment of Figure 1a, this alternating sequence of wide projections 40b and narrow projections 40a envisages one wide projection 40b for every two narrow projections 40a, but in the context of the invention further embodiments are included, not specifically illustrated in the figures, wherein the wide projections 40b and the narrow projections 40a alternate according to different sequences, or wherein there is only one type of longitudinal projection, or wherein there are more than two types of longitudinal projections that differ from each other in the values of the respective transverse dimensions.

Figures 1a, 1b and 1c refer to an embodiment of the polymeric scaffold 10 with a support structure 20 that comprises a single support layer 30. This embodiment is therefore referred to as "single-layer". In a preferred but not limitative embodiment, the total thickness of the single layer of the support 30 is in the range of 40-250 µm.

In the experimental part that follows, the preparation of two specific embodiments of the single-layer polymeric scaffold 10 is described in detail, which differ from each other in their dimensions d, L and W₁ of the cavities 41 and which, in both cases, have resulted in being effective in the tissue engineering applications concerned.

In Figure 2 an alternative embodiment is illustrated, designated as "sandwich-form", wherein the support structure 20 of the polymeric scaffold 10 comprises a first and a second support layer 30 between which an intermediate layer 33 is interposed, also made of polymeric material. According to this "sandwich-form" embodiment, the first and the second support layers 30 have cavities 41, respectively facing opposite sides of the support structure 20. The polymeric material, which the intermediate layer 33 is composed of, is preferably a biodegradable biological polymer, such as gelatin. This particular embodiment has also been shown to be effective in the tissue engineering applications concerned.

Figure 2 also shows cells C, preferably cardiac stem cells, housed inside the cavities 41 of the polymeric scaffold 10 of the invention which, consequently, after implantation, will be able to exert its cardioregenerative effects thanks to the presence of these cells that will multiply and differentiate, giving rise to new functional cardiac tissue. Of course, all the embodiments of the polymeric scaffold 10 that fall within the scope of the present invention are configured to house cardiac stem cells or other cell types effective in the regeneration of cardiac tissue.

A further particularly preferred embodiment of the polymeric scaffold 10 of the invention, which is not specifically illustrated in the figures, envisages the inclusion within the support layer 30 (see Figures 1a-1c) or of the support layers 30 and/or the intermediate layer 33 (see Figure 2) of a cardioprotective active pharmaceutical ingredient, such as adenosine or cyclosporin A, suitable for protecting cardiac tissue from damage by reperfusion. In this case the polymeric scaffold 10 will behave as a "drug reservoir", configured for releasing the pharmaceutical ingredient *in situ* according to the required times and quantities, and therefore able to exert cardioprotective effects at the same time as the cardioregenerative effects. This embodiment is particularly innovative, because, as far as the inventors are aware, polymeric scaffolds for use in tissue engineering applications, specifically cardiac tissue, equipped with cardioprotective properties against reperfusion damage, together with cardioregenerative properties, have not previously been described.

In this embodiment it is preferred that, among their constituent materials, the support layer or layers 30 and/or the intermediate layer 33 include a biological polymer capable of dissolving over time when implanted *in loco,* for example gelatin. Gelatin is a substance widely known and used in the pharmaceutical industry, where the correct choice and correct dosage can be selected by those skilled in the art, and allow adjustment of the timing and rate of release of the active ingredient.

In an even more preferred embodiment of the polymeric scaffold 101 of the invention, which is described in detail in the experimental section which follows, the active pharmaceutical ingredient is not directly included in the polymeric matrix of the scaffold but is encapsulated within nanoparticles, preferably of the core-shell type, which are in turn included in the support layer 30 or in the support layers 30 and/or intermediate layer 33. This embodiment allows the obtainment of an extended release of the active pharmaceutical ingredient over time.

With the object of obtaining completely biodegradable devices that respond to the main necessary requirements including, controlled porosity, homogeneity from a chemical point of view and satisfactory mechanical properties, the polymeric scaffolds subject of the present invention were prepared by means of two different preparation techniques: phase inversion by immersion in a non-solvent, and phase inversion by controlled evaporation of solvent mixtures. Both preparation techniques have been optimized by evaluating the effect of a large repertoire of parameters (including composition, concentrations of the polymeric solutions, the nature and quantity of solvent and non-solvent, temperature and duration of the individual phases) on the characteristics of the final products. In the experimental part that follows, several preparation examples of polymeric scaffolds are provided that fall within the scope of the present invention, using the two preparation techniques mentioned above. Of course the technical disclosure provided in these specific preparation examples are applicable, in general, to the preparation of any polymeric scaffold falling within the scope of the invention.

The experimental part that follows further illustrates the preparation of various specific embodiments of the polymeric scaffold of the invention, including single-layer, sandwich-form and drug reservoir embodiments, as well as the preparation and use of core-shell nanoparticles suitable for encapsulating the cardioprotective active pharmaceutical ingredient used in the scope of the invention. The study results of the mechanical, physico-chemical and morphological characterization are also described, conducted on various embodiments of the polymeric scaffold of the invention, as well as the results of biological tests carried out with the purpose of checking the biocompatibility, the cardioregenerative abilities and the cardioprotective capacities, in particular regarding the damage by reperfusion. This experimental part is provided for purely illustrative purposes and should not be interpreted in a limiting sense in any way of the scope of the invention as defined by the attached claims.

### EXPERIMENTAL PART

Examples 1-6 describe the preparation of polymeric matrices having different compositions and using different preparation techniques. The polymeric matrices prepared as described in examples 1-6 were subsequently used for the production of polymeric scaffolds as described in example 7.

### Example 1: preparation of PLGA/gelatin matrices

Experiments were carried out for the optimization of the method of mixing PLGA solutions in a suitable solvent, in particular dichloromethane or chloroform, and aqueous solutions of gelatin. The parameters taken into consideration were: concentration of the starting solutions, the weight ratio between the components, selection of the solvents, and method of drying (casting or lyophilization).

### Preparation by means of phase inversion by immersion in a non-solvent

PLGA solutions were prepared in dimethyl sulfoxide (DMSO) in concentrations from 1 to 5% w/v. The solutions were spread on a flat glass support or microfabricated substrate using a blade with a controlled horizontal motion, and subsequently immersed in an inversion bath consisting of a solution of water/DMSO (1/1 v/v) containing gelatin in variable concentrations (1%, 2%, 5% and 10% w/v) for 20 hours. By this method, the biological polymer is introduced into the porous structure of the membrane during the inversion phase. At the end of the inversion process, the obtained membranes were dried in a ventilated stove at 37°C +/- 1°C for 24 hours.

### Preparation by phase inversion by means of controlled evaporation of solvent mixtures

Mixtures containing two solutes (PLGA and gelatin) and three solvents were prepared according to volumetric ratios set depending on the phase diagram of the ternary mixtures.

Polymeric solutions of PLGA were prepared, in concentrations equal to 1% and 5% w/v in dichloromethane or chloroform, and acetone or acetonitrile. Aqueous solutions of gelatin were added to these, either neutral or acidified with acetic acid at 0.5% v/v, in concentrations equal to 1% and 5% w/v. The volume of the gelatin solution was varied by adding a sufficient quantity of solute in order to obtain different weight ratio percentages of the PLGA/gelatin system (95/5, 85/15, 75/25, 70/30).

### Example 2: preparation of PHBHV/gelatin matrices

The methods of preparation of these matrices were also optimized, with the object of obtaining polymeric scaffolds having high and interconnected porosity, homogeneous from a chemical point of view and characterized by a stable structure with appropriate mechanical properties. The PHBHV/gelatin-based matrices were prepared by means of a number of preparation techniques based on phase inversion by controlled evaporation of solvent mixtures and subsequent casting or lyophilization procedures. Matrices of PHBHV/gelatin were prepared in different percentage compositions: 95/5, 90/10, 85/15, 80/20 and 75/25 (w/w).

### Preparation by controlled evaporation of the solvent mixture.

### Example A

PHBHV solutions were prepared in chloroform, in concentrations equal to 1% or 5% w/v. To these solutions, aqueous solutions of gelatin were added, in concentrations equal to 1% or 5%. The quantity of gelatin solution introduced depends on the composition ratio of the bioartificial system that is required and, in particular, systems were prepared using this technique with the following percentage compositions of PHBHV/gelatin (w/w): 95/5, 90/10, 85/15, 80/20 and 75/25. Following the mixing, the solutions were stirred vigorously to allow the dispersion of the biological component, and subsequently placed in glass Petri dishes or microfabricated plates and placed in a ventilated stove (T = 25°C +/-1°C, or 30°C +/-1°C) for two hours, or freeze-dried.

### Example B

Based on data from the phase diagram of two ternary mixtures of solvents, mixtures were prepared containing the two solutes and three solvents (first solvent: dichloromethane or chloroform, second solvent: acetone or acetonitrile, third solvent: water). Solutions of PHBHV in dichloromethane or chloroform were prepared with concentrations equal to 1%, 2%, 3%, 4% and 5%. To these solutions, the second solvent was added, and finally the aqueous solution of gelatin, the concentration of which depends on the quantity of solute that is required in the final bioartificial system.

### Example 3: preparation of PDS/gelatin matrices

The PDS and gelatin-based bioartificial materials were obtained from solutions of PDS at 10% w/v, by introducing a quantity of gelatin equal to 5% w/w of PDS. Volumes of 4 ml of the solutions thus obtained were placed in glass Petri dishes (Φ = 5.5, volume 4 ml) or microfabricated plates, the solution was thickened for half an hour on the plate heated to 80°C, then the sample was immersed in a first inversion bath, consisting of a mixture of H₂O/DMSO (50/50 v/v) for one hour, and then in a second inversion bath, consisting of H₂O for one hour. Finally, the non-solvent was eliminated by casting in a ventilated stove at 40°C.

### Example 4: preparation of PDS/gellan gum matrices

PDS/gellan gum-based matrices were prepared by means of phase inversion by controlled evaporation of solvents or phase inversion by immersion in a non-solvent followed by casting or lyophilization.

### Preparation by controlled evaporation of a mixture of solvents

Warmed solutions of PDS in DMSO were prepared (at 60°C). Following the complete solubilization of the polymer, gellan gum was added in a sufficient quantity in order to obtain a PDS/gellan gum bioartificial system with a 90/10 (w/w) percentage composition. Finally, chloroform was added in a sufficient quantity in order to reach the DMSO/chloroform percentage ratio in a volume equal to 1/2. The solution thus obtained was placed in a glass petri dish or PDMS plate and maintained at a temperature of 55°C until complete evaporation of the solvents.

### Preparation by means of phase inversion by immersion in a non-solvent

### Example A

PDS solutions were prepared (at 6% and 10% w/v) in DMSO, along with gellan gum solutions in DMSO, and were mixed together in a weight ratio of 90/10. The obtained mixture was dispensed into Petri dishes or plates that had been placed on a hotplate at 50°C for half an hour in order to increase their viscosity. The mixture was then introduced, in some tests after freezing at -20°C, into an inversion water bath for 2 hours.

### Example B

A second method envisaged the use of an inversion bath consisting of a mixture of 50/50 water/DMSO for an hour, followed by a second bath consisting of water only for an hour. At the end, the preparation has a homogeneous, whitish appearance, and is fairly resistant.

The use of a blade with a controlled horizontal motion allows the homogenous depositing of the initial 90/10 PDS/gellan gum mixture with a controlled thickness. Tests carried out by varying the thickness of the deposited mixture have facilitated the application of a correspondence between this value and the final thickness of the membrane after lyophilization. For example, if the layer deposited before the inversion phase is 500 µm, the final thickness of the membrane is about 200 µm.

### Example 5: preparation of PDS/collagen matrices

PDS/collagen-based matrices were produced by means of phase inversion techniques according to two different methodologies: controlled evaporation of the solvents and immersion in non-solvent. PDS/collagen matrices were prepared in percentage compositions equal to 95/5, 90/10, 80/20 w/w.

### Preparation by means of controlled evaporation of the solvents

PDS solutions were prepared in mixtures of 2/1 v/v chloroform/DMSO at concentrations of 1%, 2%, 5% w/v, under constant stirring at 70°C. The solutions were then deposited on Petri dishes or plates and placed on a hotplate at 40°C. The membranes obtained were chemically modified by means of a crosslinking agent (carbodiimide and/or succinimide) and subsequently immersed in different solutions with variable concentrations of collagen (0.1%-1% w/v) at physiological pH for 12 hours in order to obtain the compositions mentioned above.

### Preparation by non-solvent immersion

PDS solutions were prepared in DMSO at concentrations of 3% and 10% w/v, under constant stirring at 70°C. The solutions were then deposited on glass petri dishes placed on a hotplate at 70°C to increase their viscosity. The dishes were then placed in a coagulation bath that consisted of two phases, the first consisted of a 50/50 water/DMSO mixture for one hour at room temperature, and the second consisted of double-distilled water for one hour at room temperature. Finally, the matrices thus obtained were subjected to the elimination of the non-solvent by means of casting at 40°C.

Subsequently, the membranes were chemically modified by a crosslinking agent and immersed in variable collagen solutions (0.1% to 1% w/v) at physiological pH for 12 hours in order to obtain the compositions mentioned above.

### Example 6: preparation of PDS/hyaluronic acid matrices.

PDS/hyaluronic acid (HA)-based matrices were prepared by phase inversion techniques and subsequent casting methods. PDS/HA systems were prepared with variable compositions in the range of percentage compositions of 80/20 - 40/60 (w/w).

Solutions were prepared of the activated ester of PDS by chemical derivatization (acidic hydrolysis and subsequent addition of a carbodiimide-based crosslinking agent) in DMSO, of concentrations equal to 10% w/v. The solutions deposited on dishes or plates were subjected to a first thickening phase on a 70°C hotplate, to increase the viscosity of the system. The systems were then placed in a coagulation bath that consisted of two phases (50/50 water/DMSO mixture for one hour and water only for the second hour, at room temperature) and subjected to the elimination of the non-solvent by casting at 40°C.

Aqueous solutions of HA were added to the porous PDS-based membranes, chemically functionalized in order to obtain PDS/HA ionic interaction bioartificial systems having variable weight compositions.

The preparation method was conducted under constant stirring at room temperature for 6 hours.

### Example 7: production of the polymeric scaffolds

The polymeric matrices prepared as described in examples 1-6 were used for the production of polymeric scaffolds according to the present invention. To this end, the polymeric solutions were deposited on silicon molds derived from a silicon master obtained by means of soft lithography. The molds have geometric characteristics on their surface in accordance with the present invention, designed to mimic the anatomical features of the myocardium.

A series of production drawings obtained by CAD systems (e.g. AutoCAD Mathematical) were made according to the arrays of cavities and projections mentioned above, in a wide range of sizes from 1x1 cm to 6x3 cm, suitable for cellular assays and *in vivo* implantation.

The CAD models were applied to the soft lithography technique for the production of the silicon master. A series of molds in polydimethylsiloxane (PDMS) were prepared using a Sylgard 184 Silicone Elastomer Kit product (Dow Corning Corporation) . Initially, a mixture of monomer and crosslinking agent, in a 10:1 weight ratio, was prepared by gentle mixing of the two components for 10 minutes. The steps of mixing and polymerization were conducted in a darkroom to avoid inhibition of the catalytic activity. The mixture was deposited on the silicon master and placed in a stove for 24 hours. The PDMS molds are equipped with vertical outer edges so that the volume of the solution to be deposited is contained within the mold. With this technique, scaffolds of PLGA/gel, PHBHV/gel, and PDS were made, of which the cardioregenerative ability was demonstrated. The scaffolds are characterized by a surface provided with rectangular cavities and a total thickness in the range of 40-250 µm. The depth d of the holes is in the range of 20-200 µm. The cavities have a longitudinal dimension L in the range of 350-600 µm and a transverse dimension W₁ in the range of 30-150 µm. There are wide and narrow longitudinal linear projections that have, respectively, a transverse dimension W₂ in the range of 50-160 µm and a transverse dimension W₃ in the range of 20-120 µm. The table below shows the dimensions expressed in microns (µm) measured with reference to three specific embodiments of the polymeric scaffold of the invention obtained by the above-described method.

| | Cavity | | Linear projections | | Total thickness | |
|---|---|---|---|---|---|---|
| | L | W₁ | d | W₂ | W₃ | |
| Type 1 PHBHV/gel | 400 | 30 | 20 | 160 | 120 | 70 |
| Type 2 PHBHV/gel | 485 | 85 | 40 | 60 | 25 | 100 |
| PLGA/gel | 350 | 60 | 15-20 | 60 | 30 | 40 |

The stem cells that were seeded on the single-layer scaffolds produced as previously described (see biological tests described below) have facilitated the demonstration that the combination of the particular chemical composition and the particular geometry of the polymeric scaffolds of the invention favors the colonization and differentiation toward the cardiac lineage. Indeed, neither the material alone nor the microstructures alone (as investigated by the inventors for similar microstructures based on other polymeric materials) are able to produce a similar cardioinductive effect.

Of particular relevance were the mechanical characterization results. After having calculated the actual geometric section, the elastic moduli values of the polymeric scaffolds were measured both in the longitudinal and in the transverse directions, given the anisotropic nature of the microfabricated structure. In particular, the elastic modulus value measured in the longitudinal direction is significantly greater than the value measured in the transverse direction, in agreement with the anisotropy of the biomatrix structure designed to replicate the characteristics of the cardiac tissue. In addition, the elastic modulus values recorded in an aqueous environment were lower than the values recorded in the dry tests, and closer to, in terms of absolute value, the corresponding value of the native myocardium.

A biodegradation study of the polymeric scaffolds was also carried out for a total time of 6 months. Three distinct means of degradation were considered, namely double-distilled water, PBS (phosphate buffered saline) and a culture medium (DMEM), which allows the best simulation of the *in vitro* and *in vivo* conditions. Regarding the 95/5 PHBHV/gelatin system, the pH analysis of the incubation medium revealed almost constant pH values, in a physiological range, throughout the entire period. This result suggests two important interpretations: a) the PHBHV/gelatin scaffold is stable and does not produce high amounts of degradation products, the stability of the structure is therefore reflected in the stability of the pH, b) the quantity, albeit low but detectable by the weight loss tests, of the degradation products does not alter the pH conditions. This result indicates that the PHBHV/gelatin scaffold does not induce an acidosis-induced inflammatory process *in vivo.*

Regarding the 70/30 PLGA/gelatin system, the pH analysis of the incubation medium revealed stable pH values when measured in the buffer solutions, PBS and DMEM, while a reduction in pH was confirmed after just 15 days in water. This result is due to the faster degradation rate of the PLGA-based system, as confirmed by the gravimetric tests. However, greater pH stability was observed for the PLGA/gelatin system in culture medium, a condition closer to the physiological one, compared to the other two media, with reduced percentages of weight loss and pH values never below 6 even after complete degradation.

From a morphological point of view, the results of the degradation study highlight that the scaffolds of the invention retain the geometric characteristics of the surface, without significant changes at various times of the biodegradation process, in order to guarantee the ability of the materials to drive the regenerative processes over a prolonged period. The aspect that distinguishes the three different types of scaffolds tested is the rate of biodegradation, so the choice of which biomatrix should be used will mainly be related to the surgical methods and times of the implantation, and especially to the speed of regrowth of the natural tissue at the affected area.

### Example 8: production of drug-reservoir scaffolds

By way of example, the most appropriate method of producing multilayer PLGA/gelatin scaffolds consists of the deposition of intermediate layers of aqueous solutions of gelatin at concentrations of 2-10% w/v. In detail, a gelatin solution was interposed between two microfabricated layers produced as previously described and all held together by means of die-casting at the edges. To carry out the die-casting, metal molds are arranged in a square- or rectangular-frame shape with profile dimensions chosen according to the size of the biomatrix. The molds are heated to a predetermined temperature (around 50°C for the PLGA, and 90°C for the PHBHV) and positioned at the edges of the multilayer. The method of die-casting allows the maintenance of the adhesion between the layers even after contact with the physiological environment and the consequent release of gelatin. The multilayer device offers a series of advantages: higher resistance to suture, increased section and improved mechanical properties, and the possibility to chemically or physically incorporate bioactive molecules (for example adenosine) to obtain a rapid and efficient release system.

The multi-layer scaffolds were functionalized with adenosine as a cardioprotective agent. Three different functionalization strategies with the cardioprotective drug were identified to carry out separately or in combination with each other, namely: direct loading in the support layers, direct loading in the gelatin-based intermediate layer, loading into the support and/or intermediate layers following prior encapsulation in core-shell nanoparticles. The functionalization of the polymeric scaffolds subject of the invention with the cardioprotective agent was carried out in order to achieve two fundamental objectives, namely to load the maximum quantity of adenosine or other cardioprotective agent into the biomatrix in a solubilized form, and to ensure an immediate and substantial release in a very short time period (from the initial minutes up to 24 hours). The encapsulation in nanoparticles allows the obtainment of additional protection from damage by reperfusion, as it results in a low-magnitude release of the active pharmaceutical ingredient, which is prolonged over time.

Preliminary evaluations of the solubility of adenosine in aqueous solutions containing gelatin were also carried out by increasing the temperature of the solutions. The solubility limit was estimated to be equal to 10 mg/ml, slightly above the limit reported in the literature of adenosine in water, which corresponds to 8 mg/ml.

By way of example, the preparation method of a multilayer scaffold consisting of two support layers based on 70/30 PLGA/gelatin, and an intermediate layer obtained from an aqueous solution of 10% gelatin w/w is described below. The multilayer scaffold was loaded with adenosine both in the support layers and in the intermediate layer. The total quantity of adenosine loaded into the scaffold is 17.6 mg for a device having a surface area of 10 cm² and a total thickness of 600±50 microns.

### Production of PLGA/gelatin/adenosine single-layer drug reservoir scaffolds

For the preparation of a single-layer scaffold of PLGA/gelatin functionalized with adenosine, the method described previously was used with the following modifications. Adenosine was dissolved in double-distilled water to a concentration of 10 mg/ml at a temperature of 50°C. Predefined quantities of gelatin were added to the solution of adenosine, and then the aqueous mixture of gelatin/adenosine was added to the PLGA solution in dichloromethane and acetone or acetonitrile. For example, 1 g of gelatin was added to 10 ml of adenosine solution (10mg/ml) and aliquots of this solution were added to a solution of PLGA in dichloromethane and acetone or acetonitrile. The deposition of the bioartificial mixture was conducted as follows: a) 1 ml of the PLGA/gelatin/adenosine solution was deposited on a microfabricated PDMS substrate (of a surface area of 10 cm²); after 10 minutes at room temperature, a second deposition was repeated with 1 ml of solution, b) slow and controlled drying, c) detachment of the single layer from the substrate.

The single-layer PLGA/gelatin/adenosine scaffolds obtained were also used to prepare multilayer scaffolds loaded with adenosine, as described in example 10 below.

### Production of PLGA/gelatin/adenosine multilayer drug reservoir scaffolds

As previously described, 1 g of gelatin was added to 10 ml of an adenosine solution of 10 mg/ml concentration. The gelatin/adenosine solution was deposited on a single layer on the cavity- and projection-free side. Initially, a volume of 500 µl of gelatin/adenosine solution was homogeneously distributed over the entire surface, and then a second aliquot of 500 µl was deposited and distributed on the same surface. A second microfabricated membrane was assembled leaving the side with the profiled surface free. Finally, the two layers were adhered by gentle die-casting conducted, as previously described, at 50°C. The drug reservoir systems were dried and stored in a freezer before *in vitro* and *in vivo* biological characterization.

In a similar manner to that described for the PLGA/gelatin scaffolds, multilayer drug reservoir scaffolds were prepared using PHBHV, PDS and gelatin or other biological polymers. The assembly method of the individual layers envisaged higher die-casting temperatures, of up to 80-90°C, given the greater heat resistance of these materials.

### Sterilization of the final scaffold loaded with adenosine

The sterilization was conducted by introducing the scaffold to a dish containing an aqueous solution of 70% ethanol for 15 minutes, followed by five repeat washes with fresh solution. Subsequently, the solution was removed, and the scaffold was dried completely under a sterile laminar flow hood. The final step consisted of irradiating the samples with UV rays for 15 minutes on both sides.

### Release test, morphological and physico-chemical characterization of the final scaffolds

On the 70/30 PLGA/gelatin multilayer drug reservoir scaffolds functionalized with adenosine, release tests were also conducted after sterilization, and after having verified the possible loss of the drug in the ethanol solution. The results of the HPLC analysis reported a partial release of adenosine in the ethanol solution used for sterilization, however, most of the loaded adenosine remained inside the biomatrix (values higher than 70%). The final sterilized scaffolds were placed in tubes containing double-distilled water at 37°C under constant shaking, and for predetermined intervals (1,2,10,30,60 minutes and 24 hours). The supernatant was collected and analyzed by HPLC analysis. An appreciable release was evident for all times analyzed, from 1 minute to 1 hour. The quantity of adenosine recorded after 24 hours of release corresponds to 43% compared to the quantity initially introduced, a high percentage in accordance with the initial objectives and in line with the adenosine doses administered intraluminally.

Observations were carried out by scanning electron microscopy (SEM) of the final multilayer drug reservoir scaffolds functionalized with adenosine. After 24 hours in double-distilled water, the presence of voids was observed that were not initially present, originating from the dissolution of the adenosine and gelatin at the level of the individual support layers and intermediate layers, while the geometric characteristics of the surface of the scaffolds (cavities and projections) were preserved, almost unchanged. These SEM observations indicate a complete release of both gelatin and adenosine after 24 hours. The results obtained confirm the validity of the loading method adopted, showing that the scaffold assembled as such forms an optimal platform for a rapid and sustained release of a cardioprotective agent. Once the release function has been completed for reducing reperfusion injury, the scaffold can then continue to carry out the other function of inducing the regenerative processes of the cardiac tissue.

The complete release of adenosine was also confirmed by means of FT-IR Chemical Imaging analysis.

The scaffolds were also tested for suturability and gripping to the delivery device in view of the *in vivo* implant. The tests were successfully completed.

### Example 9: Functionalization of nanoparticles with SDF-1α and adenosine

As indicated above, the multi-layer drug reservoir scaffolds of the present invention were also functionalized with a cardioprotective agent encapsulated in core-shell nanoparticles, with the object of obtaining a sustained release of the drug.

### Production of P(BMA-co-(PEG)MEMA) nanoparticles for the release of adenosine and SDF-1α

Nanoparticles were produced with an internal reserve of P(BMA-co-(PEG)MEMA) for the loading of active ingredients to be incorporated in the scaffolds of the invention. These are nanosystems able to combine the nanometric dimensional characteristics with the internal reserve structure i.e. equipped with an internal cavity that allows the maximization of the efficiency of encapsulation. Such nanoparticles were used for the incorporation of both adenosine and of the factor SDF-1α.

The preparation of core-shell polymeric nanoparticles envisages the use of a "sacrificial" polymeric core, consisting of synthetic nanoparticles, which are subsequently used as template for the synthesis of a cross-linked surface layer that forms the shell. Subsequently, the core is removed by using suitable solvents, thus obtaining nanoparticles with a reserve, also denominated nanocapsules.

According to the present example, the core used is made of polymethylmethacrylate (PMMA), while the outer shell was obtained by the copolymerization of butyl methacrylate (BMA) and polyethylene glycol methyl ether methacrylate with an average molecular weight of 300 g/mol ((PEG)MEMA)300 in the presence of a crosslinker, trimethylolpropane trimethacrylate (TRIM). The reaction product was recovered, ultracentrifuged (at 14000 rpm) and subjected to removal of the core. The nanocapsules obtained were then loaded by adsorption with adenosine or SDF-1α.

The table below reports the materials used for the synthesis of the nanocapsules of P(BMA-co-(PEG)MEMA) specifying the purpose of use.

| Name | Chemical formula | Characteristics | Use |
|---|---|---|---|
| MONOMERS: | | | |
| Methyl methacrylate (MMA) | 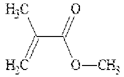 | Sigma Aldrich®, density 0.936 g/ml (25°C) | Monomer for synthesis of the core |
| Butyl methacrylate (BMA) | 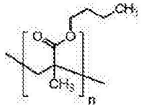 | Sigma Aldrich®, molecular weight 142.20 g/mol, density 0.894 g/ml (25°C). | Co-monomers for synthesis of the shell |
| polyethylene glycol methyl ether methacrylate ((PEG)MEMA) | 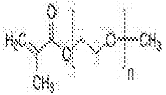 | Sigma Aldrich®, average molecular weight 300 g/mol and a density of 1.05 g/ml (25 °C) | |
| Trimethylolpropane trimethacrylate (TRIM) | 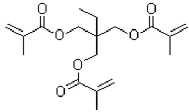 | Sigma Aldrich®, molecular weight 338.40 g/mol, density 1.060 g/cm³ (25°C) | Crosslinking agent |

| REDOX COUPLE: | | | |
|---|---|---|---|
| Ammonium persulfate | (NH4)₂S₂O₈ | Carlo Erba®, purity 98% | Initiators, redox couple |
| Sodium metabisulfite | Na₂O₅S₂ | Carlo Erba®, purity 97% | |

| SOL VENTS: | | | |
|---|---|---|---|
| Dichloromethane | 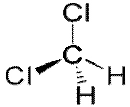 | Carlo Erba®, purity of 99.5% | Used as a solvent for the sacrificial core (extraction). |
| 2-propanol | 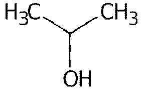 | Sigma Aldrich®, purity of 99% | Used for the purification of the nanocapsules. Removing a possible fraction of PBMA homopolymer present in the reaction product. |

| OTHER MATERIALS: | | | |
|---|---|---|---|
| Sodium dodecyl sulfate (SDS) | 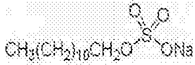 | Sigma Aldrich® | Surfactant used for the emulsion polymerization. |
| Phosphate buffer (PBS) | | Sigma Aldrich® | Used to evaluate the drug release and absorption kinetics. |
| Adenosine | 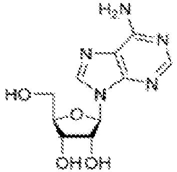 | Sigma Aldrich®, molecular weight 267.24 g/mol, purity of 99% | Signaling mole-cule |
| *SDF-1**α*** | | Santa Cruz Biotechnology, Inc., molecular weight 35 kDa, 68 amino acids of human origin. | Signaling molecule |

Below, the three steps of preparation of the reserve nanoparticles are described:
synthesis of the core (polymer: PMMA)
synthesis of the shell(polymer: P(BMA-co-(PEG)MEMA)) extraction of the core

### Synthesis of the core (PMMA)

The particles to be used as the core were directly synthesized in the form of nanoparticles using MMA as the starting monomer. The reaction medium is composed of a 60/40 (v/v) mixture of water and ethanol, containing sodium dodecyl sulfate as the surfactant. For the initiator of the radical reaction, a sodium metabisulfite- and ammonium persulfate-based redox couple was used.

The reaction apparatus consists of a three-necked flask with a 1000 ml capacity immersed in an oil bath maintained at a temperature of 37°C for the entire duration of the reaction (3h) by a thermocouple; the reaction is conducted under constant stirring by means of the use of a blade stirrer (50 W Heidolf RZR 2020), set to 1600 rpm.

The table below reports the components used to carry out the synthesis of the PMMA core:

| Reagents | Quantity |
|---|---|
| MMA | 0.25 mol |
| SDS | 2g |
| Ammonium persulfate | 0.003 mol |
| Sodium metabisulfite | 0.003 mol |
| H₂O | 350 ml |
| Ethanol | 150 ml |

The synthesis was carried out by adding water and SDS into the flask 250 ml. Once a homogeneous emulsion was obtained, 150 ml of ethanol and the MMA monomer were added. The redox couple was previously solubilized in 100 ml of H₂O at 37°C; the solution was then added to the reaction phase; the latter was degassed with dry nitrogen for a few minutes, in order to remove any oxygen that might inhibit the start of the reaction. The synthesis was conducted by spinning at a speed of 1600 rpm at 37°C for 2 hours. At the end, the reaction product was placed in a ventilated stove at 37°C to remove water and ethanol. The nanoparticles obtained were subjected to washing with double-distilled water at a temperature of 80°C to remove residues of unreacted monomer and the surfactant, and then dried again. Finally, the particulate material was crushed with a mortar to remove any aggregates and analyzed using a scanning electron microscope.

### Synthesis of the shell (P(BMA-co-(PEG)MEMA))

The copolymerization of two monomers (PEG)MEMA and BMA in a molar ratio of 20/80 was carried out. The crosslinker TRIM was added to the reaction mixture, in quantities equal to 20% relative to the moles of monomer, in order to maintain the copolymeric structure formed around the core even after its extraction. The same conditions of temperature and spinning speed were used as those described for the synthesis of the core, as well as the same reaction apparatus. The composition of the reaction mixture is shown in the table below.

| | |
|---|---|
| BMA | 0.1 mol |
| (PEG)MEMA | 0.025 mol |
| TRIM | 0.025 mol |
| SDS | 2g |
| Ammonium persulfate | 0.0012 mol |
| Sodium metabisulfite | 0.0012 mol |
| Double-distilled water | 350 ml |
| Ethanol | 150 ml |

Initially, 250 ml of water and SDS was introduced into the reactor; followed by the addition of ethanol and 5 g of previously purified, dried and crushed PMMA particles.

Then the monomers and the redox couple were added, previously dissolved in 100 ml of water at 37°C. Finally, the reaction mixture was degassed with nitrogen for 10 minutes and maintained for two hours at room temperature (37°C) and constant agitation (600 rpm). At the end of the reaction, the product obtained was recovered and placed in a ventilated stove at 37°C to remove the solvents, water and ethanol.

### Extraction of the core

The third step consisted in the elimination of the PMMA core in order to obtain a "hollow" particle formed only of the cross-linked copolymer P(BMA-co-(PEG)MEMA).

Dichloromethane was selected as the extraction solvent because it is able to dissolve the PMMA without dissolving the copolymer of the shell. The nanoparticles were immersed in a 20 ml volume of solvent and agitated at room temperature for 15 minutes. Elimination of the dichloromethane-PMMA solution was carried out by filtration, after which the recovered nanoparticles were placed in a second solvent, 2-propanol, for 15 minutes in order to remove any residual PBMA homopolymer. After filtration the particles were placed in a ventilated stove at 37°C to remove residual solvent, and once dried, they were subjected to washing in double-distilled water at 80°C, and finally dried again in a ventilated stove. At the end of the procedure, the yield of extracted particles relative to the non-extracted particles initially used corresponds to about 44%.

### Method of adenosine loading into the nanoparticles

Prior to loading, the nanoparticles were preconditioned in PBS for 18 hours, by placing the materials in contact with a PBS solution at room temperature. For the adenosine loading, three solutions of adenosine in PBS at different concentration were prepared: 2 mg/ml (solution A), 1 mg/ml (solution B), 0.5 mg/ml (solution C). At least three samples of nanoparticles were prepared for each loading solution. The adsorption process of adenosine on the nanoparticles was conducted at room temperature for a total time of 4 hours. At one-hour intervals, 0.5 ml of each solution in contact with the particles was taken and analyzed, after each collection, 0.5 ml of the respective adenosine solutions was replenished. After each collection, the samples were vigorously stirred using a vortex mixer for 15 minutes, after which, they were centrifuged for 15 minutes at 14000 rpm. At the end of the loading process, the polymeric samples were separated from the residual absorption solution by centrifuging for 30 minutes at 14000 rpm and eliminating the remaining supernatant. Finally the samples loaded as such were placed in a ventilated stove at 37°C for 12 hours.

### Method of SDF-1α loading

At the end of the conditioning process, carried out as described above, the particles were placed in contact with 1 ml of SDF-1α solution in PBS at a concentration of 1µg/ml. At regular time intervals for a total period of two hours, aliquots of 100 µl of the absorbing medium were taken, at each collection, the medium was replenished with 100 µl of fresh solution containing the active ingredient. The samples were stirred vigorously using a vortex mixer for 15 minutes, followed by centrifugation for 15 minutes at 14000 rpm. At the end of centrifugation, samples were subjected to quantitative analysis by HPLC in order to evaluate the kinetics of absorption of the stromal factor. At the end of the loading procedure, the polymeric samples were separated from the residual absorption solution by centrifugation for 30 minutes at 14000 rpm and removing the remaining supernatant. Finally, the nanoparticulate samples containing SDF-1α were placed in a ventilated stove at 37°C for 12 hours .

### BIOLOGICAL TESTS

The study of the interactions that occur between the scaffold and the cellular component represents a field of high scientific and technological interest. Assays performed *in vitro* using cell culture allow information to be obtained, related to the biological compatibility of the studied materials, making these assays indispensable, both in the understanding of the mechanisms of cell-material interactions, and improving the biological response obtained, prior to proceeding with *in vivo* assays.

Highly sensitive and specific, the *in vitro* techniques allow the assessment of parameters such as, for example, the surface morphology, porosity, chemical composition of the materials in question and the direct evaluation of how these materials affect key aspects of cellular activity: adhesion, proliferation, metabolic activity, vitality. The studies were followed by the analysis of phenotypic traits acquired from the stem cells after culture on the biomatrices, because the ability of the biomatrices to direct the cells toward the cardiac phenotype is fundamental.

Reperfusion, even if it is necessary for reducing the damage caused by the absence of oxygen and nutrients, causes further damage in its perimeter area affected by necrosis, damage that can however be limited. For this reason it was decided to functionalize the scaffolds with a cardioprotective factor, to act in the very initial post-infarction moments, limiting the damage by reperfusion as much as possible, then focusing on the protection of the still-healthy tissue.

### Example 10: Evaluation of biocompatibility

### Cells deemed relevant for seeding on the scaffolds MSC: mesenchymal stem cells

Adult mesenchymal stem cells (MSC) are a population of multipotent cells that reside primarily in the bone marrow, although these cells can also be isolated from tissues such as the placenta, umbilical cord, lungs, liver, skeletal muscle, fat, and dental pulp. MSCs are characterized by being able to differentiate easily, *in vitro,* into bone, cartilage and adipose tissue. Recently, an important paracrine role has been observed at the myocardial level, which suggested a possible transdifferentiation in the cardiomyocyte sense, making these cells extremely interesting in the field of cardiac regeneration. These paracrine effects include the secretion of factors that can attenuate the apoptosis of endogenous cardiomyocytes and endothelial cells, can promote angiogenesis, and can activate resident cardiac stem cells.

Finally, a further mechanism whereby bone marrow stem cells can contribute to myocardial repair is through the maintenance of the pool of cardiac-specific stem cells after injury. The bone marrow stem cells localizing in the myocardium after injury undergo phenotypic changes to adapt to the resident cardiac cell phenotype. These cells could contribute to the long-term endogenous repair capacity of the heart.

### CSC: resident cardiac stem cells

In recent years, evidence has emerged that demonstrates a certain regenerative capacity of the myocardial tissue. Recent studies have shown that the heart has a reserve of small cells expressing stem cell markers (c-kit⁺, OCT 3/4 CD90) and equipped with telomerase activity, typical of cells capable of replication. The resident cardiac stem cells (CSC) are directed toward a differentiation pathway, which leads to the formation of cardioblasts and subsequently to cardiomyocytes. Cardiac stem cells are characterized by a low rate of replication, they give rise to highly proliferating "lineage restricted" progenitors that become "committed" precursors toward cardiac differentiation, and finally reach growth arrest and terminal differentiation. The identification of these cells explains the observation of mitotic events interspersed in the adult myocardium, the frequency of which increases dramatically in response to stress (e.g. cardiac failure, pressure overload). By studying these populations, it is desired that the prospect of an autologous cell therapy may be achieved, carried out with cardiac stem cells, with all the possible benefits that this therapeutic approach can deliver.

### METHODS

### Cell seeding on the biomatrices

The seeding is carried out on biomatrices placed in separate wells of a 24-well plate. To improve the adhesion capacity it is necessary to seed the cells in a drop of about 50 µl of culture medium, this means that the cells adhere to the matrix, rather than at the bottom of the well to which they would adhere if the drop was not localized only on the surface of the matrix. The ability of the matrix to retain the drop thus influences the adhesion ability of the cells to the matrix itself.

The plate containing the matrices is subsequently placed, with delicate movements so that the drop is not moved from the substrate, in an incubator at 37°C, 5% CO₂ and a water vapor-saturated atmosphere, to allow cell adhesion. After about 3 hours (time of adhesion) a further 450 µl of complete medium is added for each biomatrix in order to cover it; the plate is again placed in the incubator.

The concentration of cells to be seeded, for the cell adhesion, is always calculated as the number of cells per cm² and thus takes into account the size of the areas used for seeding which, in the case of the substrates used by us, is 1 cm² . To evaluate the optimal concentration of cells, seeding with increasing concentrations of cells was carried out, from 5000 to 30000 for each matrix. Based on the results obtained in this way, it was possible to better evaluate the concentration of cells to be seeded according to the different objectives of each experiment.

### Cell adhesion, proliferation and viability on the biomatrices

The presence of a particular extracellular environment contributes to determining if and when the cell divides. Unsuitable stimuli from the external environment can also lead to cell death. This can occur by necrosis or apoptosis. The control of cell proliferation and of their survival on biomaterials is crucial to the regeneration of the tissues. In order to evaluate the proliferation on biomatrices, a first cell passage is necessary, that is, the evaluation of the ability of the cells to adhere, and this is carried out in a standard way after 24 hours from seeding. To achieve efficient regeneration it is fundamental that the cells adhere to the matrix: several experiments seem to confirm that the cells adhere better to microstructured substrates compared to smooth surfaces. Indeed, microstructured substrates manage to trap the cells penetrated within them, which present a greater number of focal adhesions.

### Indirect CellTiter-Blue™ Reagent tests

This assay uses a fluorimetric/colorimetric growth indicator to detect cellular metabolic activity. It consists of a buffered solution containing a highly purified molecule, resazurin, which is dark blue in color and has little intrinsic fluorescence. The metabolically active cells possess enzymes that are able to reduce resazurin to another molecule, resorufin, which is pink and is highly fluorescent, with an excitation maximum of 579 nm and an emission maximum of 584 nm. The resorufin is released into the culture medium and, thanks to its fluorescence, can be detected with the use of a microplate reader. The measurement of fluorescence is easy to obtain due to the fact that the molecule used is able to spontaneously leave the cells, without damaging them, and can then be repeated on the same samples at different days from seeding.

The quantification of the number of cells is based on the extrapolation of the data from a calibration curve. Furthermore, the cells remain viable and therefore the treatment can be repeated several times on the same biomatrix at different timings, thus allowing long-term information to be obtained. Precisely this important advantage, added to the relative ease of performing the test, meant that this test was chosen for our adhesion and proliferation experiments.

To obtain data concerning the cell adhesion to the matrix it is necessary to seed the cells in parallel both on the matrices to be tested (3D) and directly on the 24 well-plates (in 2D). The cells in 2D are seeded with known and increasing concentrations, this allows the creation of a calibration curve: every known number of seeded cells (x) will have a relative fluorescence value (y). Entering the values obtained from the samples for the calibration, into a graph, a curve can be obtained from which a linear regression line is created, which allows extrapolation of the number of cells (x) effectively adherent to the matrix of which the fluorescence value (y) is known. Each cell line will have its own reference curve because each cell type has its own intrinsic metabolic activity.

The test to evaluate the cell adhesion is carried out on the day after seeding (after 24 hours) to allow the cells to adhere properly to the substrate. The incubation time is defined by the color change of the medium during the incubation with the CellTiter Blue. When it is considered that the timing of incubation is sufficient for a good sensitivity, reading with a microplate reader can then be carried out.

To evaluate the proliferation it is necessary to use the same timing of incubation with the CellTiter-blue for all the experiments carried out on the same sample, in this way the increase of fluorescence detected will be solely due to an increase in the number of cells. It is therefore possible to have indirect data on the number of cells present by extrapolating the number of cells of the sample relative to the calibration curve seeded and read at 24 hours.

It is therefore possible to normalize all subsequent fluorescence values relative to the first value, which was obtained at 24 hours: this therefore provides a value indicative of the increase in fluorescence and thus of the increase in the number of cells. The first fluorescence value is set as equal to the value 1, if there is an increase in the number of cells, values will be greater, if instead there is a reduction in the number of cells, values will be between 0 and 1. Expressing these values in a graph, a histogram is obtained that allows comparison, in a very intuitive way, of the proliferation that has taken place on different biomatrices, this piece of data is, however, independent from the quantity of cells that had initially adhered.

### Tests with Calcein-AM

Calcein-AM is a vital dye for obtaining information on the health of the cells, protracted over time. In this way, the viability of the cells can be evaluated, which present an intense green coloring when viable, and at the same time semi-quantitative data on the metabolic activity can be obtained, since the molecule is cut by cytoplasmic esterases present when the metabolism is active. In addition, calcein colors the entire cytoplasm highlighting the cell morphology and allowing the observation of the cells on the supports at different days after seeding.

### RESULTS

The experimental data obtained by the present inventors allow the observation of how the cell adhesion is facilitated if the substrate presents a microstructuring: the geometry of the microstructuring is also able to facilitate the ordered arrangement of the cells.

The percentage of cell adhesion on the matrices based on PHBHV, PLGA, PDS, and biological polymers that have microstructuring, varies between 40 and 70%, while the assay performed on the same non-microstructured matrices has revealed a percentage of adhesion only detectable in a few cases.

It was also demonstrated that the adhesion on matrices is favored in the presence of a natural component: the data obtained reveal that the PDS support without a natural component is able to adhere a smaller number of cells, when compared to supports where a natural component is present.

The proliferation is favored in the presence of the microstructuring; the data highlight a higher proliferation on microstructured matrices subject of the invention, compared to the same non-microstructured matrices.

The 3D systems, based on bioartificial polymeric materials, subject of the present invention, have been demonstrated to be excellent supports for cell adhesion and proliferation, in particular those that have a geometric structure specifically designed to mimic the extracellular matrix of the cardiac wall. It is interesting to note that the presence of the natural component can positively influence the capacity of cell adhesion.

### Example 11: Evaluation of the cardioinductivity

The environment in which the stem cells are cultured is very important because it is able to influence the cell phenotype, i.e. the set of features manifested by the cells, defined by specific markers characteristic of each cell type. In particular, it was observed that, for the initial orientation toward a determined differentiated phenotype, the type of support to which the stem cells adhere and on which they grow is fundamental, both for its physico-chemical characteristics and for its geometry.

The cell growth experiments carried out by us on biomatrices have highlighted a decrease in cell proliferation between day 11 and day 15 after seeding. A possible initiation of differentiation was therefore hypothesized, which has caused us to specifically investigate the possible changes in the cell phenotype.

After carefully assessing the most important stem cell and differentiation markers, c-kit was selected as a marker of staminality and GATA 4 as a marker of early differentiation toward cardiomyocytes. The techniques chosen to study the markers were qPCR and immunofluorescence: the former is a very sensitive technique that is able to detect even very small changes in gene expression between 2D- and matrix-seeded cells. To confirm that possible variations in transcript expression also correspond to changes in protein expression, we carried out immunofluorescence experiments.

From the results obtained from the analysis of gene expression of transcription factors, it emerges that the particular geometry, in association with the bioartificial polymeric materials used in the invention, is capable of directing the mesenchymal stem cells (MSC) toward an early initial differentiation to cardiomyocytes, highlighted by the significant increase of the marker GATA 4, at the same time conferring to them a loss of staminality, with a decrease of the marker c-kit.

Resident cardiac stem cells (CSC) cultured on biomatrices, demonstrate significant activity of some late differentiation markers, genes whose expression gives rise to important structural and functional proteins.

The stem cell markers, however, have a variable trend: this is probably an indication that the CSCs are a mixed population already directed toward a path of differentiation, therefore they have already lost some of the stem cell characteristics. The analysis carried out on markers of early differentiation once again highlighted the variable data regarding the transcription factor GATA 4.

More effective results are obtained when markers are studied for the expression of genes encoding structural and functional proteins, important for cardiac function, such as connexin 43, responsible for the electrical connections between the cells, troponin C, of fundamental importance in the skeletal muscle excitation-contraction phase and α-SMA, with functional characteristics of the contractile type. Indeed, the CSCs significantly increase the expression of two of these markers analyzed, emphasizing the ability of this type of stem cell, if appropriately stimulated, to increase their already partial differentiation. For this reason as well, the expression of GATA 4, a very early marker of differentiation, does not reach significant levels, while the transcription factor MEF2C undergoes a clear downregulation, not present in hMSC cultured on the matrix, highlighting that there is a drive toward cardiac differentiation and not simply toward striated muscle differentiation.

These gene expression data, both for the MSCs and the CSCs, were confirmed on the corresponding proteins by immunofluorescence assays and confocal microscopy.

The results obtained on the biomatrices have demonstrated their precise geometry, which resembles the structure of the ventricular wall, and in association with the bioartificial polymeric materials used in the present invention, can induce the stem cells toward an initial cardiomyocyte differentiation. The analysis of the chosen markers has highlighted that MSCs, after 15 days of culture, are able to decrease the stemness characteristics and acquire early cardiac phenotypic characteristics. Resident cardiac stem cells, CSCs, when kept in the same conditions as MSCs, are able to express markers of an even more advanced cardiomyocyte differentiation stage, because they are stem cells that are already in a more advanced stage of differentiation: the physical stimulus of the matrices therefore pushes them to a further step ahead in the differentiation process.

### Example 12: Evaluation of the cardioprotective capacity against reperfusion injury

In order to protect the still-healthy tissue present in the early post-myocardial infarction stages, it is important to act upon limiting the damage caused by reperfusion. Indeed, the reopening of the coronary artery causes damage caused precisely by the new blood supply that leads to high inflammation and oxidative stress. During the reperfusion phase there is, in addition to cellular calcium overload, a release of free radicals (first of all the superoxide anion) by the endothelium of the coronary vessel and by the granulocytes that are adherent to the walls of the coronary endothelium. These events lead to the opening of the mitochondrial transition pore, resulting in activation of the process of cell death by apoptosis.

The myocardial ischemic fiber produces adenosine, a factor that determines coronary vasodilation in the event of further requests for oxygen, therefore in conditions of hypoxia as well. Adenosine has both a vaso-dilatory effect and a protective effect on the myocardium, and results in a reduction of metabolism, with a consequent decrease in oxygen demand. In addition, adenosine increases the opening of potassium channels and this leads to an accelerated repolarization with reduction of the plateau, and consequently reduces the time available to the calcium for entering the cell. This means that calcium overload is prevented, resulting in reduction of ischemic damage.

The action of adenosine is also evident at the mitochondrial level: it can in fact limit the apoptotic processes activated by the suffering cardiomyocytes. The cardioprotective molecule is able to limit the opening of the mitochondrial transition pore, localized at some points of contact between the two mitochondrial membranes, preventing the release of protons which would lead to an alteration of the membrane potential, resulting in entry of liquids into the mitochondria, breaking of the outer membrane and release of apoptotic factors (cytochrome C and caspase-9).

There is also activation of the anti-apoptotic protein Bcl 2 that is expressed through two independent pathways activated by adenosine: the first involves the phosphorylation of PKCε protein which in turn phosphorylates and activates ERK, while the second pathway leads to the phosphorylation of AKT which in turn phosphorylates and activates GSK3β. Phospho-ERK and GSK3β can then move to the mitochondria and act on mitochondrial Bcl-2, inhibiting the apoptotic process activated by the suffering cells after reperfusion.

For these reasons, it was decided to functionalize the biomatrices with adenosine, in order to strengthen the protective effect that the heart already activates endogenously, to a small degree. Adenosine must, therefore, begin to be released in the first few moments after implantation and in this way the damage caused by the new blood supply to the injured area will be limited.

The experiments conducted *in vitro* have demonstrated that the biomatrices functionalized with the cardioprotective factor adenosine are able to protect the mesenchymal stem cells subjected to hypoxia for 72 hours, followed by 3 hours of recovery in normoxia, a situation that mimics the condition of ischemia followed by reperfusion. This is confirmed both by the ability of cells to maintain a good viability, and from the analysis of the proteins involved in the process of inhibition of the apoptotic process.

The CellTiter Blue assay, which demonstrated the viability of the cells was carried out prior to the hypoxic stimulus and during the 3 hours of recovery: the fluorescence measurements obtained during the 3 hours of recovery were normalized according to the measurements obtained before the hypoxic stimulus, in order to evaluate the maintenance of the viability. The data obtained show that the MSCs, in the presence of the functionalized biomatrix, suffer limited damage when compared to the same cells in the same conditions, but without the matrix. Furthermore, the viability is greater compared to the same MSCs that only received adenosine.

Subsequent analyzes have enabled the evaluation of the activation of the pathways associated with the process of cardioprotection in cultured cells with a functionalized biomatrix, or rather the presence of some of the anti-apoptotic effector proteins involved in the process, such as ERK and AKT in their phosphorylated form. The results obtained show how these proteins in cells cultured with a biomatrix functionalized with adenosine, display a phosphorylation of proteins comparable to the same proteins of cells cultured in the presence of adenosine in the culture medium, thus demonstrating the effectiveness of the release of the cardioprotective molecule.

## Claims

1. A polymeric scaffold for cardiac regeneration (10), comprising a support structure (20) comprising at least one support layer (30) **characterized in that** it is formed of a polymeric material selected from the group consisting of poly(lactic-co-glycolic) acid, poly(3-hydroxybutyric-co-3-hydroxyvaleric) acid and poly(dioxanone), possibly in association with a biological polymer, the support layer (30) being further **characterized in that** it has a surface on which a plurality of cavities (41) is formed, arranged in a regular lattice, said cavities (41) being defined by an array of longitudinal linear projections (40a, 40b) and by an array of transverse linear projections (40c), respectively intersecting each other.

2. A polymeric scaffold for cardiac regeneration (10) according to claim 1, wherein said cavities (41) have a depth d in the range of 20 to 100 µm.

3. A polymeric scaffold for cardiac regeneration (10) according to claim 1 or 2, wherein said cavities (41) have a longitudinal dimension 1 within the range of 350 to 600 µm and a transverse dimension w₁ in the range of 30 to 150 µm.

4. A polymeric scaffold for cardiac regeneration (10) according to any one of claims 1 to 3, wherein said array of longitudinal linear projections (40a, 40b) comprises an alternating sequence of wide projections (40b) and narrow projections (40a), wherein the transverse dimension w₂ of the wide projections (40b) is in the range of 50 to 160 µm and the transverse dimension w₃ of the narrow projections (40a) is in the range of 20 to 120 µm, with the proviso that w₂> w₃.

5. A polymeric scaffold for cardiac regeneration (10) according to claim 4, wherein said alternating sequence envisages one wide projection (40b) for every two narrow projections (40a).

6. A polymeric scaffold for cardiac regeneration (10) according to any one of claims 1 to 5, wherein said cavities (41) have an essentially rectangular shape.

7. A polymeric scaffold for cardiac regeneration (10) according to any one of claims 1 to 6, wherein said biological polymer is selected from the group consisting of gelatin, hyaluronic acid, collagen and gellan gum.

8. A polymeric scaffold for cardiac regeneration (10) according to any one of claims 1 to 7, wherein said support structure (20) comprises a first and a second of said support layers (30) and an intermediate layer (33) of polymeric material interposed between said first and second support layers (30), said first and second supporting layers (30) having cavities (41) respectively facing opposite sides of the support structure (20).

9. A polymeric scaffold for cardiac regeneration (10) according to claim 8, wherein said polymeric material of the intermediate layer (33) is a biological polymer.

10. A polymeric scaffold for cardiac regeneration (10) according to claim 9, wherein said polymeric material of the intermediate layer (33) is gelatin.

11. A polymeric scaffold for cardiac regeneration (10) according to any one of claims 1 to 10, also comprising a cardioprotective active pharmaceutical ingredient.

12. A polymeric scaffold for cardiac regeneration (10) according to claim 11, wherein said cardioprotective active pharmaceutical ingredient is adenosine or cyclosporine A.

13. A polymeric scaffold for cardiac regeneration (10) according to claim 11 or 12, wherein said active pharmaceutical ingredient is included in the intermediate layer (33) and/or in the support layer or layers (30) .

14. A polymeric scaffold for cardiac regeneration (10) according to any one of claims 11 to 13, wherein said active pharmaceutical ingredient is encapsulated in core-shell nanoparticles.

15. A polymeric scaffold for cardiac regeneration (10) according to any one of claims 1 to 14, also comprising a plurality of cardiac stem cells (C) housed in said cavities (41).

## Patentansprüche

1. Ein Polymergerüst für kardiale Regenerierung (10), mit einer Stützstruktur (20) mit mindestens einer Stützschicht (30), **dadurch gekennzeichnet,**
**dass** dieses aus einem Polymerwerkstoff gebildet ist, der aus der Gruppe bestehend aus Poly(lactic-co-glycolid)-Säure, Poly(3-hydroxybutyric-co-3-hydroxyvaleric)-Säure und Polydioxanon ausgewählt wird, optional in Verbindung mit einem biologischen Polymer,
wobei die Stützschicht (30) weiter **dadurch gekennzeichnet ist,**
**dass** sie eine Oberfläche aufweist, an der eine Vielzahl von Vertiefungen (41) ausgebildet sind, die in einem regelmäßigen Gitter angeordnet sind, wobei die Vertiefungen (41) durch eine Reihe von linearen Erhebungen in Längsrichtung (41a, 41b) und durch eine Reihe von linearen Erhebungen in Transversalrichtung (40c) definiert sind, die sich jeweils gegenseitig schneiden.

2. Ein Polymergerüst für kardiale Regenerierung (10) nach Anspruch 1, **dadurch gekennzeichnet, dass** die Vertiefungen (41) eine Tiefe d im Bereich von 20 bis 100 µm aufweisen.

3. Ein Polymergerüst für kardiale Regenerierung (10) nach Ansprüchen 1 oder 2, **dadurch gekennzeichnet, dass** die genannten Vertiefungen (41) eine Längsausdehnung I im Bereich von 350 bis 600 µm und eine Querausdehnung w₁ im Bereich von 30 bis 150 µm aufweisen.

4. Ein Polymergerüst für kardiale Regenerierung (10) nach einem der Ansprüche 1 bis 3, **dadurch gekennzeichnet, dass** die genannte Reihe linearer Erhebungen in Längsrichtung (40a, 40b) eine alternierende Folge von breiten Erhebungen (40b) und schmalen Erhebungen (40a) aufweist, wobei die Breitenausdehnung w₂ der breiten Erhebungen (40b) im Bereich von 50 bis 160 µm und die Breitenausdehnung w₃ der schmalen Erhebungen (40a) im Bereich von 20 bis 120 µm liegt, mit der Bedingung, dass w₂ > w₃ ist.

5. Ein Polymergerüst für kardiale Regenerierung (10) nach Anspruch 4, **dadurch gekennzeichnet, dass** die genannte alternierende Folge eine breite Erhebung (40b) für jeweils zwei schmale Erhebungen (40a) vorsieht.

6. Ein Polymergerüst für kardiale Regenerierung (10) nach einem der Ansprüche 1 bis 5, **dadurch gekennzeichnet, dass** die genannten Vertiefungen (41) eine im Wesentlichen rechteckige Form aufweisen.

7. Ein Polymergerüst für kardiale Regenerierung (10) nach einem der Ansprüche 1 bis 6, **dadurch gekennzeichnet, dass** das biologische Polymer ausgewählt ist aus der Gruppe bestehend aus Gelatine, Hyaluronsäure, Collagen und Gellan-Gummi.

8. Ein Polymergerüst für kardiale Regenerierung (10) nach einem der Ansprüche 1 bis 7, **dadurch gekennzeichnet, dass** die Stützstruktur (20) eine erste und eine zweite Stützschicht (30) und eine Zwischenschicht (33) aus einem Polymerwerkstoff zwischen der ersten und zweiten Stützschicht (30) aufweist, wobei die erste und zweite Stützschicht (30) Vertiefungen (41) aufweisen, die sich jeweils auf entgegengesetzten Seiten der Stützstruktur (20) befinden.

9. Ein Polymergerüst für kardiale Regenerierung (10) nach Anspruch 8, **dadurch gekennzeichnet, dass** der Polymerwerkstoff der Zwischenschicht (33) ein biologisches Polymer ist.

10. Ein Polymergerüst für kardiale Regenerierung (10) nach Anspruch 9, **dadurch gekennzeichnet, dass** der Polymerwerkstoff der Zwischenschicht (33) Gelatin ist.

11. Ein Polymergerüst für kardiale Regenerierung (10) nach einem der Ansprüche 1 bis 10, **dadurch gekennzeichnet, dass** dieses einen kardioprotektiven aktiven pharmazeutischen Inhaltsstoff aufweist.

12. Ein Polymergerüst für kardiale Regenerierung (10) nach Anspruch 11, **dadurch gekennzeichnet, dass** es sich bei dem kardioprotektiven aktiven pharmazeutischen Inhaltsstoff um Adenosin oder Cyclosporin A handelt.

13. Ein Polymergerüst für kardiale Regenerierung (10) nach Ansprüchen 11 oder 12, **dadurch gekennzeichnet, dass** der aktive pharmazeutische Inhaltsstoff in die Zwischenschicht (33) und/ oder in die Stützschicht oder -schichten (30) eingeschlossen ist.

14. Ein Polymergerüst für kardiale Regenerierung (10) nach einem der Ansprüche 11 bis 13, **dadurch gekennzeichnet, dass** der aktive pharmazeutische Inhaltsstoff in Kern-Schale-Nanopartikel eingekapselt ist.

15. Ein Polymergerüst für kardiale Regenerierung (10) nach einem der Ansprüche 1 bis 14, **dadurch gekennzeichnet, dass** es eine Vielzahl kardialer Stammzellen (C) in den Vertiefungen (41) aufweist.

## Revendications

1. Échafaudage polymère pour régénération cardiaque (10), comprenant une structure de support (20) comprenant au moins une couche de support (30) **caractérisée en ce qu'**elle est formée d'un matériau polymère choisi dans le groupe constitué de l'acide poly(lactique-co-glycolique), de l'acide poly(3-hydroxybutyrique-co-3-hydroxyvalérique) et de la poly(dioxanone), éventuellement en association avec un polymère biologique, la couche de support (30) étant en outre **caractérisée en ce qu'**elle a une surface sur laquelle est formée une pluralité de cavités (41), agencées en réseau régulier, lesdites cavités (41) étant définies par un ensemble de saillies linéaires longitudinales (40a, 40b) et par un ensemble de saillies linéaires transversales (40c), se croisant respectivement.

2. Échafaudage polymère pour régénération cardiaque (10) selon la revendication 1, dans lequel lesdites cavités (41) ont une profondeur d comprise dans la plage allant de 20 à 100 µm.

3. Échafaudage polymère pour régénération cardiaque (10) selon la revendication 1 ou 2, dans lequel lesdites cavités (41) ont une dimension longitudinale l comprise dans la plage allant de 350 à 600 µm une dimension transversale w₁ comprise dans la plage allant de 30 à 150 µm.

4. Échafaudage polymère pour régénération cardiaque (10) selon l'une quelconque des revendications 1 à 3, dans lequel ledit ensemble de saillies linéaires longitudinales (40a, 40b) comprend une séquence alternée de saillies larges (40b) et de saillies étroites (40a), où la dimension transversale w₂ des saillies larges (40b) est comprise dans la plage allant de 50 à 160 µm et la dimension transversale w₃ des saillies étroites (40a) est comprise dans la plage allant de 20 à 120 µm, à condition que w₂ > w₃.

5. Échafaudage polymère pour régénération cardiaque (10) selon la revendication 4, dans lequel ladite séquence alternée prévoit une saillie large (40b) pour chaque deux saillies étroites (40a).

6. Échafaudage polymère pour régénération cardiaque (10) selon l'une quelconque des revendications 1 à 5, dans lequel lesdites cavités (41) ont une forme essentiellement rectangulaire.

7. Échafaudage polymère pour régénération cardiaque (10) selon l'une quelconque des revendications 1 à 6, dans lequel ledit polymère biologique est choisi dans le groupe constitué de la gélatine, de l'acide hyaluronique, du collagène et de la gomme gellane.

8. Échafaudage polymère pour régénération cardiaque (10) selon l'une quelconque des revendications 1 à 7, dans lequel ladite structure de support (20) comprend une première et une deuxième desdites couches de support (30) et une couche intermédiaire (33) de matériau polymère interposée entre lesdits premier et deuxième couches de support (30), lesdites première et deuxième couches de support (30) ayant des cavités (41) faisant face respectivement à des côtés opposés de la structure de support (20).

9. Échafaudage polymère pour régénération cardiaque (10) selon la revendication 8, dans lequel ledit matériau polymère de la couche intermédiaire (33) est un polymère biologique.

10. Échafaudage polymère pour régénération cardiaque (10) selon la revendication 9, dans lequel ledit matériau polymère de la couche intermédiaire (33) est la gélatine.

11. Échafaudage polymère pour régénération cardiaque (10) selon l'une quelconque des revendications 1 à 10, comprenant également un ingrédient pharmaceutique actif cardioprotecteur.

12. Échafaudage polymère pour régénération cardiaque (10) selon la revendication 11, dans lequel ledit ingrédient pharmaceutique actif cardioprotecteur est l'adénosine ou la cyclosporine A.

13. Échafaudage polymère pour régénération cardiaque (10) selon la revendication 11 ou 12, dans lequel ledit ingrédient pharmaceutique actif est inclus dans la couche intermédiaire (33) et/ou dans la ou les couche(s) de support (30).

14. Échafaudage polymère pour régénération cardiaque (10) selon l'une quelconque des revendications 11 à 13, dans lequel ledit ingrédient pharmaceutique actif est encapsulé dans des nanoparticules à noyau-enveloppe.

15. Échafaudage polymère pour régénération cardiaque (10) selon l'une quelconque des revendications 1 à 14, comprenant également une pluralité de cellules souches cardiaques (C) logées dans lesdites cavités (41).
